# EUROPEAN PATENT APPLICATION

(11) **EP 3 275 501 A1**
(43) Date of publication of application: **31.01.2018**
(21) Application number: 17157700.0
(22) Date of filing: 23.02.2017
(51) Int. Cl.: A61M 37/00, A61G 15/14, A61G 15/16, A61C 1/00, A61B 50/20

(54) **HOUSING ASSEMBLY INCLUDING CRADLE ASSEMBLIES CONFIGURED TO SUPPORT TATTOO MACHINES**

(30) Priority: 29.07.2016 US 201615223090
(71) Applicant: Hofung, Weizhong, Markham, Ontario L6C 9P3 (CA)
(72) Inventor: Hofung, Weizhong, Markham, Ontario L6C 9P3 (CA)
(74) Representative: Oliveira Lourenço, Nuno Miguel

(57) **Abstract**

An apparatus includes cradle assemblies (102) configured to respectively receive and support tattoo machines (900), in which the tattoo machines are configured to be electrically powered. A housing assembly (104) is positioned relative to the cradle assemblies. The housing assembly is configured to selectively provide electrical power to the tattoo machines, in which the tattoo machines are to be respectively received and supported by the cradle assemblies. The housing assembly is configured to receive and support the cradle assemblies.

## Description

### TECHNICAL FIELD

This document relates to the technical field of (and is not limited to) an apparatus including cradle assemblies configured to respectively receive and support tattoo machines, and a housing assembly positioned relative to the cradle assemblies (and method therefor).

### BACKGROUND

A tattoo machine (also called a tattoo gun) is a hand-held device generally used to create a tattoo, a permanent marking of the skin with indelible ink. Modem tattoo machines use electromagnetic coils to move an armature bar up and down. Connected to the armature bar is a barred needle grouping that pushes ink into the skin. Tattoo artists generally use the term "machine", or even "iron", to refer to their equipment. The word "gun" is often used but many tattoo professionals dislike it. In addition to the coiled tattoo machines there are also rotary tattoo machines, which are powered by regulated motors rather than electromagnetic coils.

### SUMMARY

It will be appreciated that there exists a need to mitigate (at least in part) at least one problem associated with the existing tattoo machines (also called the existing technology). After much study of the known systems and methods with experimentation, an understanding of the problem and its solution has been identified and is articulated as follows:

Various tattoo inking tasks need to be performed by a tattoo machine, depending on how deep the ink is to be placed on the skin, etc. In order to satisfy the various inking tasks, the tattoo machine is adjusted for power level (electrical power consumption). What is inconvenient about this arrangement is that the power level of the tattoo machine is adjusted from time to time, thereby wasting time and effort for making such power level adjustments to the tattoo machine, and sometimes, leading to inconsistent results for the tattoo artist (user of the tattoo machine) and an unsatisfied customer (the person receiving the tattoo).

What is needed is a plurality of tattoo machines, in which each machine is individually controlled and adjusted for power, such as power set-up, current limits, etc., for a particular inking task to be performed for a selected tattoo machine. However, this may be an expensive arrangement simply because each tattoo machine would require its own dedicated power supply, especially since the tattoo artist can only use one tattoo machine at a time, while the remaining tattoo machines remain idle. To counteract this disadvantage, it would be a good idea to have all of the tattoo machines powered by a single power source.

In addition, it may be a good idea to organize and store the tattoo machines that are required to remain idle and ready for easy user access, while a selected tattoo machine is powered and actively used by the tattoo artist. Therefore, it would be a good idea to have a housing assembly that is configured to (A) receive and support the tattoo machines, and (B) selectively connect electrical power to the tattoo machines. It would be a good idea, as well, to disable the electrical power to be provided or connected to the tattoo machines that remain idle, while only the active tattoo machine remains electrically powered. It would also be a good idea to have an arrangement that would accommodate relatively easier cleaning and sanitizing of the housing assembly (or components thereof) for an improved hygienic outcome.

In accordance with a preferred embodiment, each of the tattoo machines may be individually turned OFF for the case where the user desires to avoid accidentally connecting power to a selected tattoo machine.

To mitigate, at least in part, at least one problem associated with the existing technology, there is provided (in accordance with a major aspect) an apparatus. The apparatus includes cradle assemblies configured to respectively receive and support tattoo machines, in which the tattoo machines are configured to be electrically powered. A housing assembly is positioned relative to the cradle assemblies. The housing assembly is configured to selectively provide (connect) electrical power to the tattoo machines, in which the tattoo machines are to be respectively received and supported by the cradle assemblies. The housing assembly is configured to receive and support the cradle assemblies.

In accordance with a preferred embodiment, the housing assembly is configured to support the tattoo machines. The housing assembly is configured to provide individual power control for each of the tattoo machines, such as power set-up, current limits, etc., that may be required for various tattoo inking tasks to be performed by the tattoo machines.

In accordance with a preferred embodiment, pressure switches are configured to disable power to be provided or connected to a selected one of the tattoo machines, in such a way that only the selected one of the tattoo machines is provided with or is connected to electrical power while the remaining tattoo machines remain unpowered.

In accordance with a preferred embodiment, the cradle assembly is removable from the pressure switch in such a way that the cradle assembly may be sanitized. The pressure switch extends, at least in part, from the housing assembly. The advantage of this embodiment is that the apparatus may be more easily kept hygienic.

In accordance with a preferred embodiment, each of the tattoo machines may be individually turned OFF for the case where the user desires to avoid accidentally connecting power to a selected tattoo machine.

Other aspects are identified in the claims.

Other aspects and features of the non-limiting embodiments may now become apparent to those skilled in the art upon review of the following detailed description of the non-limiting embodiments with the accompanying drawings.

This Summary is provided to introduce concepts in simplified form that are further described below in the Detailed Description. This Summary is not intended to identify key features or essential features of the disclosed subject matter, and is not intended to describe each disclosed embodiment or every implementation of the disclosed subject matter. Many other novel advantages, features, and relationships will become apparent as this description proceeds. The figures and the description that follow more particularly exemplify illustrative embodiments.

### BRIEF DESCRIPTION OF THE DRAWINGS

The non-limiting embodiments may be more fully appreciated by reference to the following detailed description of the non-limiting embodiments when taken in conjunction with the accompanying drawings, in which:
FIG. 1 depicts a front perspective view of an embodiment of an apparatus including a combination of cradle assemblies configured to support a respective tattoo machine, and a housing assembly;
FIG. 2 depicts a rear perspective view of an embodiment of the apparatus of FIG. 1; and
FIGS. 3 to 6 depict side views of embodiments of the apparatus of FIG. 1.

The drawings are not necessarily to scale and may be illustrated by phantom lines, diagrammatic representations and fragmentary views. In certain instances, details unnecessary for an understanding of the embodiments (and/or details that render other details difficult to perceive) may have been omitted.

Corresponding reference characters indicate corresponding components throughout the several figures of the drawings. Elements in the several figures are illustrated for simplicity and clarity and have not been drawn to scale. The dimensions of some of the elements in the figures may be emphasized relative to other elements for facilitating an understanding of the various disclosed embodiments. In addition, common, but well-understood, elements that are useful or necessary in commercially feasible embodiments are often not depicted to provide a less obstructed view of the embodiments of the present disclosure.

### LISTING OF REFERENCE NUMERALS USED IN THE DRAWINGS

- 102: cradle assemblies
- 104: housing assembly
- 106: foot-activated switch
- 108: external power supply
- 110: controller
- 112: power display unit
- 114: power-adjustment switch
- 116: power switch
- 118: pressure switch
- 120: power output
- 122: power-cord input
- 124: power cord
- 126: external power supply cord
- 128: external power supply wire
- 130: external power input
- 132: foot-switch input
- 134: foot-switch wire
- 140: stand-off portion
- 900: tattoo machines
- 902: user
- 903: foot

### DETAILED DESCRIPTION OF THE NON-LIMITING EMBODIMENT(S)

The following detailed description is merely exemplary and is not intended to limit the described embodiments or the application and uses of the described embodiments. As used, the word "exemplary" or "illustrative" means "serving as an example, instance, or illustration." Any implementation described as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure. The scope of may be defined by the claims (in which the claims may be amended during patent examination after filing of this application). For the description, the terms "upper," "lower," "left," "rear," "right," "front," "vertical," "horizontal," and derivatives thereof shall relate to the examples as oriented in the drawings. There is no intention to be bound by any expressed or implied theory in the preceding Technical Field, Background, Summary or the following detailed description. It is also to be understood that the devices and processes illustrated in the attached drawings, and described in the following specification, are exemplary embodiments (examples), aspects and/or concepts defined in the appended claims. Hence, dimensions and other physical characteristics relating to the embodiments disclosed are not to be considered as limiting, unless the claims expressly state otherwise. It is understood that the phrase "at least one" is equivalent to "a". The aspects (examples, alterations, modifications, options, variations, embodiments and any equivalent thereof) are described regarding the drawings. It should be understood that the invention is limited to the subject matter provided by the claims, and that the invention is not limited to the particular aspects depicted and described.

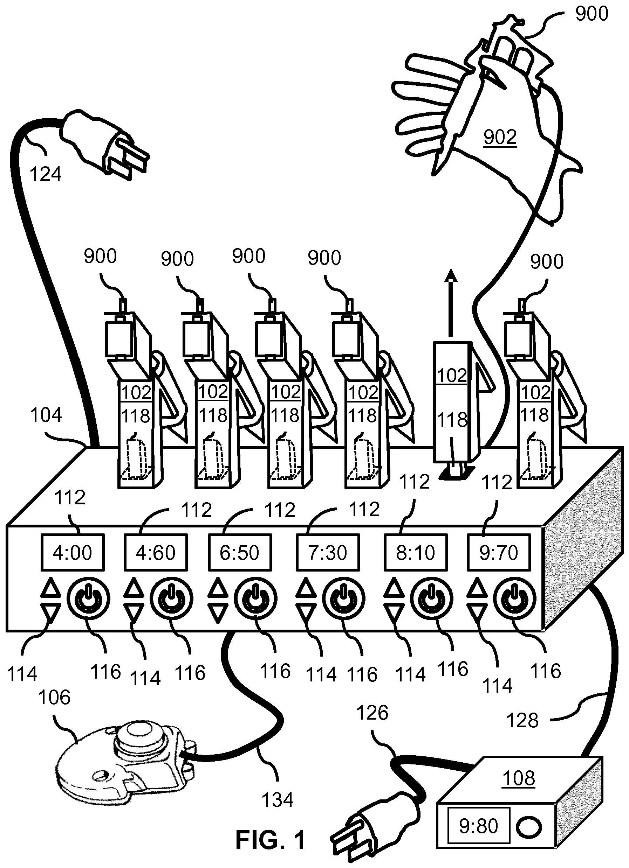

FIG. 1 depicts a front perspective view of an embodiment of an apparatus including a combination of cradle assemblies 102 configured to support a respective tattoo machine 900 (also called a tattoo gun), and a housing assembly 104.

Referring to the embodiment as depicted in FIG. 1, the apparatus includes (and is not limited to) a synergistic combination of the cradle assemblies 102 and the housing assembly 104. The cradle assemblies 102 are configured to respectively receive and support the tattoo machines 900, in which the tattoo machines 900 are configured to be electrically powered. The housing assembly 104 is positioned relative to the cradle assemblies 102. The housing assembly 104 is configured to selectively connect electrical power to the tattoo machines 900, in which the tattoo machines 900 are to be respectively received and supported by the cradle assemblies 102. The housing assembly 104 is configured to receive and support the cradle assemblies 102.

In accordance with a preferred embodiment, the housing assembly 104 is configured to support the tattoo machines 900. The housing assembly 104 is configured to provide individual power control for each of the tattoo machines, such as power set-up, current limits, etc., that may be required for various tattoo inking tasks to be performed by the tattoo machines.

In accordance with a specific embodiment, the housing assembly 104 is configured to receive and support the cradle assemblies 102. This configuration is done in such a way that the cradle assemblies 102 are spaced apart relative to each other once the housing assembly 104, in use, receives and supports the cradle assemblies 102. In addition, this configuration is also done in such a way that the tattoo machines 900, which are respectively received and supported by the cradle assemblies 102, are spaced apart from each other once: (a) the housing assembly 104, in use, receives and supports the cradle assemblies 102, and (b) the cradle assemblies 102, in use, respectively receive and support the tattoo machines 900.

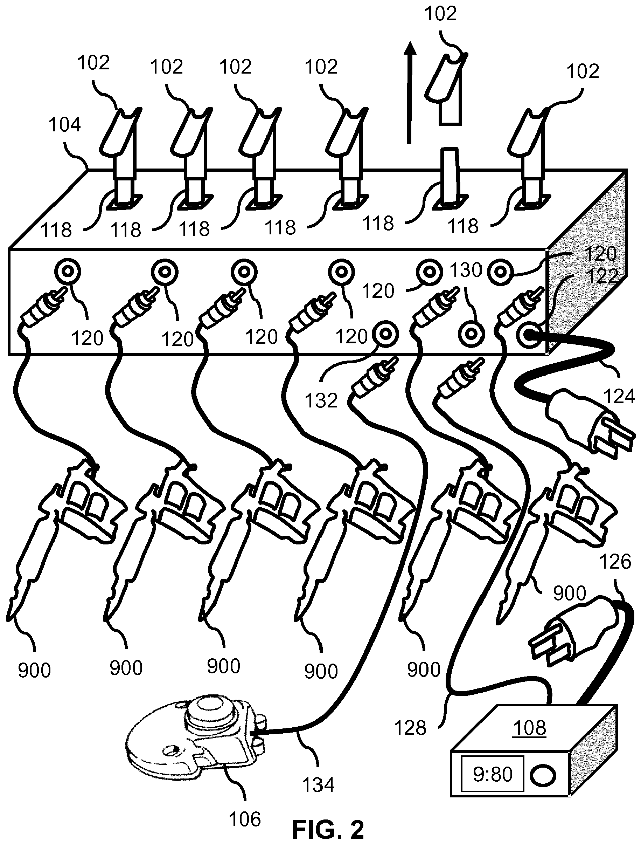

FIG. 2 depicts a rear perspective view of an embodiment of the apparatus of FIG. 1.

Referring to the embodiments as depicted in FIGS. 1 and 2, there is a quantity of six cradle assemblies 102 that are cooperative with (or installed to or coupled to) the housing assembly 104. It will be appreciated that other embodiments are arranged such that a quantity of two, three, four, five or more cradle assemblies 102 are cooperative with (installed to) the housing assembly 104.

Referring to the embodiment as depicted in FIG. 1, a user 902 (artist) is depicted as picking up one of the tattoo machines 900 from a selected cradle assembly 102, while the other tattoo machines 900 remain received by their respective cradle assemblies 102.

Referring to the embodiments as depicted in FIGS. 1 and 2, the housing assembly 104 includes pressure switches 118 (also called limit switches or position switches). The pressure switches 118 are configured to be interactive with (interfaced with) a respective cradle assembly 102.

In accordance with a preferred embodiment, the pressure switches 118 are configured to disable power to be provided or connected to a selected one of the tattoo machines 900, in such a way that only the selected one of the tattoo machines 900 is provided with or is connected to electrical power while the remaining tattoo machines 900 remain unpowered.

The pressure switches 118 are configured to: (A) detect whether a respective cradle assembly 102 is supporting a respective tattoo machine 900, and (B) detect whether the respective tattoo machine 900 has been removed from the respective cradle assembly 102. The pressure switches 118 are configured to retract further into a central interior of the housing assembly 104 in response to the respective cradle assembly 102, in use, supporting the respective tattoo machine 900. The pressure switches 118 are configured to extend away from the central interior of the housing assembly 104 in response to the removal of the respective tattoo machine 900 from the respective cradle assembly 102.

For the case where (A) electrical power is connected to the housing assembly 104 (via a power cord 124 or via an external power supply 108), and (B) the respective tattoo machine 900 remains received and supported by the respective cradle assembly 102, the pressure switch 118 is configured to not permit (disconnect) the supply of electrical power to the respective tattoo machine 900.

For the case where (A) electrical power is connected to the housing assembly 104 (via a power cord 124 or via an external power supply 108), and (B) the respective tattoo machine 900 is removed from the respective cradle assembly 102, the pressure switch 118 is configured to permit (connect) the supply of electrical power to the respective tattoo machine 900.

Referring to the embodiments as depicted in FIGS. 1 and 2, and in accordance with an option, the housing assembly 104 further includes a power-cord input 122. The power-cord input 122 is configured to be connectable with a power cord 124, which is connectable to an electrical outlet (also called a wall outlet, which is known and not depicted) mounted to a wall, etc. In this manner, the housing assembly 104 may receive electrical power form the power cord 124 that is plugged into the electrical outlet for powering the tattoo machines 900.

Referring to the embodiments as depicted in FIGS. 1 and 2, and in accordance with an option, the housing assembly 104 further includes an external power input 130. The external power input 130 is configured to be electrically connected to an external power supply wire 128. The external power supply wire 128 is configured to be electrically connected to an external power supply 108. In this manner, the housing assembly 104 may receive electrical power from the external power supply 108, which is used for powering the tattoo machines 900. An external power supply cord 126 is configured to electrically connect the external power supply 108 to an electrical outlet. An embodiment of the external power supply 108 includes the Model Number PS-C1 power supply (provided by World Wide Tattoo located in British Columbia, Canada). It will be appreciated that in accordance with an embodiment, the housing assembly 104 further includes the combination of the power-cord input 122 and the external power input 130 (for improved powering options).

Referring to the embodiments as depicted in FIGS. 1 and 2, the housing assembly 104 further includes a foot-switch input 132. The foot-switch input 132 is configured to be electrically connected to a plug of a foot-switch wire 134. The foot-switch wire 134 is configured to be electrically connected to a foot-activated switch 106. The foot-activated switch 106 is configured to: (A) turn OFF the electrical power that is provided to a selected one of the tattoo machines 900, and (B) turn OFF the electrical power in such a way that no electrical power is provided to any one of the tattoo machines 900.

Referring to the embodiment as depicted in FIG. 1, the housing assembly 104 further includes a power display unit 112 for a respective one of the tattoo machines 900 to be respectively received and supported by the cradle assemblies 102. The power display unit 112 is configured to provide an indication of the amount of power to be provided to a selected one of the tattoo machines 900. The housing assembly 104 further includes a power-adjustment switch 114 for a respective one of the tattoo machines 900 to be respectively received and supported by the cradle assemblies 102. The power-adjustment switch 114 is configured to be selectively adjusted by a user in such a way that the amount of power to be consumed by a selected tattoo machine 900 may be adjustable (either relatively higher or lower as may be needed).

The housing assembly 104 further includes a power switch 116 (ON/OFF switch) for a respective one of the tattoo machines 900 to be respectively received and supported by the cradle assemblies 102.The power switch 116 is configured to selectively turn ON or turn OFF power to be made available to a selected one of the tattoo machines 900. In this manner, each of the tattoo machines 900 can be individually turned OFF for the case where the user desires to avoid accidentally connecting power to a selected tattoo machine 900.

Referring to the embodiment as depicted in FIG. 2, the cradle assemblies 102 are configured to be removable from a respective pressure switch 118.

Referring to the embodiment as depicted in FIG. 2, the housing assembly 104 includes a power output 120. The power output 120 is configured to be electrically connected to a respective tattoo machine 900.

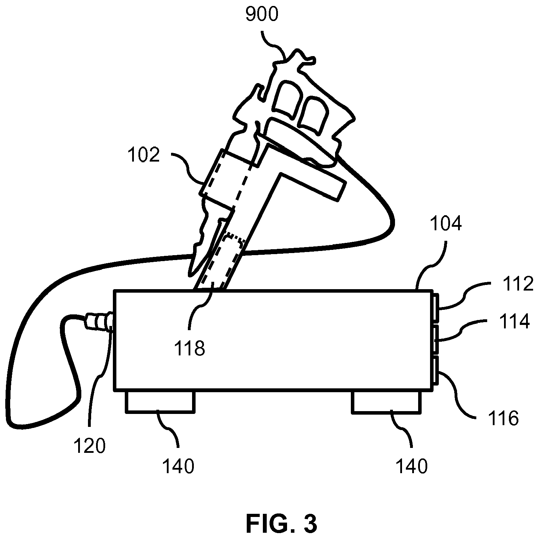

FIGS. 3 to 6 depict side views of embodiments of the apparatus of FIG. 1.

Referring to the embodiment as depicted in FIG. 3, the pressure switch 118 extends from the interior of the housing assembly 104 to an exterior of the housing assembly 104. The cradle assembly 102 is made to (is positioned to) contact the pressure switch 118 in such a way that the pressure switch 118 receives and supports the cradle assembly 102, while the cradle assembly 102 receives and supports a respective tattoo machine 900. The tattoo machine 900 is electrically connected to the power output 120 of the housing assembly 104. The housing assembly 104 includes a stand-off portion 140 (for contacting a working surface).

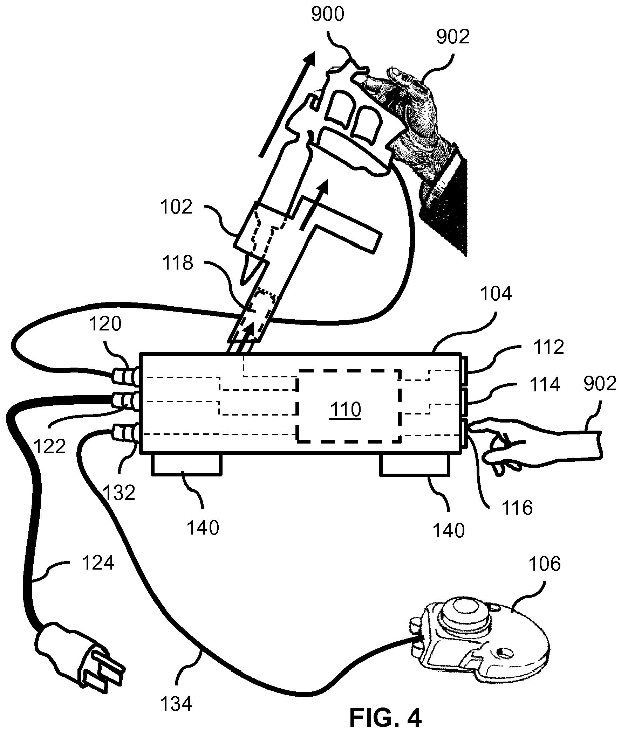

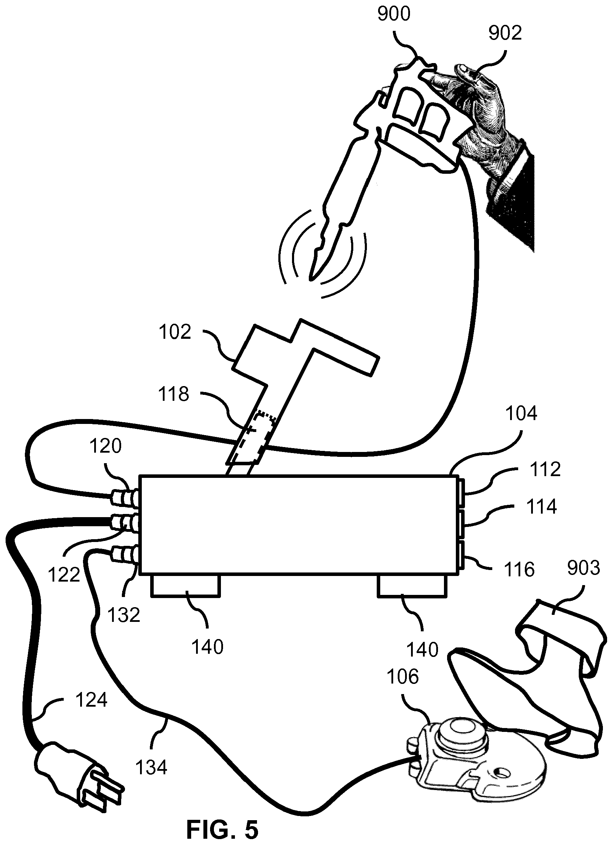

Referring to the embodiment as depicted in FIG. 4, the housing assembly 104 further includes a controller 110. The controller 110 is electrically connected to the power display unit 112, the power-adjustment switch 114, and the power switch 116 (ON/OFF switch). The controller 110 is electrically connected to the power output 120, in which the power output 120 is configured to be electrically connected to a respective tattoo machine 900. The controller 110 is configured to be electrically connectable to any one of (A) the power-cord input 122 (which is electrically connected to the power cord 124 as depicted in FIG. 5), and (B) the power output 120 (which is electrically connected to the external power supply 108). The controller 110 is electrically connected to the foot-switch input 132, in which the foot-switch input 132 is configured to be electrically connected to the foot-switch wire 134 of the foot-activated switch 106. The foot-activated switch 106 may include a simple ON/OFF switch or any equivalent thereof for making (closing) or breaking (opening) a circuit. The foot-activated switch 106 is configured to be (A) closed in response to the foot 903 of the user 902 depressing the foot-activated switch 106, which, in this case, indicates a power ON request to the controller 110 via the foot-switch wire 134), and (B) open in response to the foot 903 of the user 902 not depressing the foot-activated switch 106, which, in this case, indicates a power OFF request to the controller 110 via the foot-switch wire 134). For the case where the foot-activated switch 106 is depressed by the foot 903 of the user 902, the foot-activated switch 106, in use, provides a first indication to the controller 110 that the user desires an application of electrical power to be connected to the tattoo machine 900. For the case where the foot-activated switch 106 is not depressed by the foot 903 of the user 902, the foot-activated switch 106, in use, provides a second indication to the controller 110 that the user does not desire the application of electrical power to the tattoo machine 900. The controller 110 is electrically connected to the pressure switch 118. The tattoo machine 900 is respectively received and supported by the cradle assembly 102.

The pressure switch 118 detects that the tattoo machine 900 is received and supported by the cradle assembly 102. The pressure switch 118 is configured to transmit a first signal to the controller 110, in which the first signal indicates that the tattoo machine 900 is received and supported by the cradle assembly 102.

The controller 110 is configured to not connect electrical power to the tattoo machine 900 for the case where the controller 110 receives the first signal from the pressure switch 118 (for the case where the tattoo machine 900 is received and supported by the cradle assembly 102).

The pressure switch 118 is configured to detect whether the tattoo machine 900 has become removed from the cradle assembly 102, and to provide a second signal indicating that the tattoo machine 900 has become removed from the cradle assembly 102. The pressure switch 118 is configured to detect that the tattoo machine 900 has become removed from the cradle assembly 102, for the case where the user 902 removes the tattoo machine 900 from the cradle assembly 102, and away from the housing assembly 104. The pressure switch 118 is configured to transmit a second signal to the controller 110, in which the second signal indicates that the tattoo machine 900 has become removed from the cradle assembly 102.

Referring to the embodiment as depicted in FIG. 4, the controller 110 is configured to not connect electrical power to the tattoo machine 900 for the case where the controller 110 (A) receives the first signal from the pressure switch 118 (for the case where the tattoo machine 900 is, at least in part, received and supported by the cradle assembly 102), and (B) receives the second indication from the foot-activated switch 106 (in which the user 902 does not desire the application of electrical power to the tattoo machine 900).

Referring to the embodiment as depicted in FIG. 5, the controller 110 is configured to connect electrical power to the tattoo machine 900 for the case where the controller 110 (A) receives the second signal from the pressure switch 118 (for the case where the tattoo machine 900 is not received and supported by the cradle assembly 102), and (B) receives the first indication from the foot-activated switch 106 (in which the user desires an application of electrical power to be connected to the tattoo machine 900).

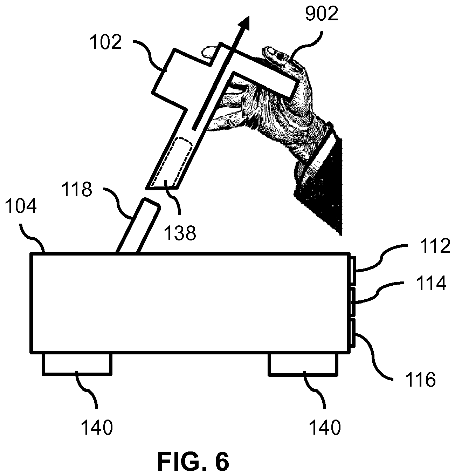

Referring to the embodiment as depicted in FIG. 6, the cradle assembly 102 is removable from the pressure switch 118 in such a way that the cradle assembly 102 may be sanitized. The pressure switch 118 extends, at least in part, from the housing assembly 104. The advantage of this embodiment is that the apparatus may be more easily kept hygienic.

It will be appreciated that the description and/or drawings identify and describe embodiments of the apparatus (either explicitly or non-explicitly). The apparatus may include any suitable combination and/or permutation of the technical features as identified in the detailed description, as may be required and/or desired to suit a particular technical purpose and/or technical function. It will be appreciated, that where possible and suitable, any one or more of the technical features of the apparatus may be combined with any other one or more of the technical features of the apparatus (in any combination and/or permutation). It will be appreciated that persons skilled in the art would know that technical features of each embodiment may be deployed (where possible) in other embodiments even if not expressly stated as such above. It will be appreciated that persons skilled in the art would know that other options would be possible for the configuration of the components of the apparatus to adjust to manufacturing requirements and still remain within the scope as described in at least one or more of the claims. This written description provides embodiments, including the best mode, and also enables the person skilled in the art to make and use the embodiments. The patentable scope may be defined by the claims. The written description and/or drawings may help understand the scope of the claims. It is believed that all the crucial aspects of the disclosed subject matter have been provided in this document. It is understood, for this document, that the phrase "includes" is equivalent to the word "comprising." The foregoing has outlined the non-limiting embodiments (examples). The description is made for particular non-limiting embodiments (examples). It is understood that the non-limiting embodiments are merely illustrative as examples.

## Claims

1. An apparatus, comprising:
cradle assemblies being configured to respectively receive and support tattoo machines, in which the tattoo machines are configured to be electrically powered; and
a housing assembly being positioned relative to the cradle assemblies; and
the housing assembly being configured to selectively provide electrical power to the tattoo machines, in which the tattoo machines are to be respectively received and supported by the cradle assemblies; and
the housing assembly being configured to receive and support the cradle assemblies.

2. The apparatus of claim 1, wherein:
the housing assembly is configured to receive and support the cradle assemblies in such a way that:
the cradle assemblies are spaced apart relative to each other once the housing assembly, in use, receives and supports the cradle assemblies; and
the tattoo machines, which are respectively received and supported by the cradle assemblies, are spaced apart from each other once:
the housing assembly, in use, receives and supports the cradle assemblies; and
the cradle assemblies, in use, respectively receive and support the tattoo machines.

3. The apparatus of any one of claim 1 and claim 2, wherein:
the housing assembly includes:
pressure switches configured to be interactive and interfaced with a respective cradle assembly.

4. The apparatus of claim 3, wherein:
the pressure switches are configured to:
detect whether the cradle assembly is supporting a tattoo machine; and
detect whether the tattoo machine has been removed from the cradle assembly.

5. The apparatus of any one of claim 3 and claim 4, wherein:
the pressure switches are configured to extend away from a central interior of the housing assembly in response to removal of a tattoo machine from the cradle assembly.

6. The apparatus of any one of claim 3, claim 4 and claim 5, wherein:
the pressure switches are configured to not permit a supply of electrical power to a tattoo machine for the case where:
electrical power is connected to the housing assembly via any one of a power cord and an external power supply; and
the tattoo machine remains received and supported by the cradle assembly.

7. The apparatus of any one of claim 3, claim 4, claim 5, claim 6, wherein:
the pressure switches are configured to permit a supply of electrical power to a tattoo machine for the case where:
electrical power is connected to the housing assembly via any one of a power cord and an external power supply; and
the tattoo machine is removed from the cradle assembly.

8. The apparatus of any one of claim 3, claim 4, claim 5, claim 6 and claim 7, wherein:
the housing assembly further includes:
a power display unit for a respective one of the tattoo machines to be respectively received and supported by the cradle assemblies; and
the power display unit configured to provide an indication of an amount of power to be provided to a selected one of the tattoo machines.

9. The apparatus of any one of claim 3, claim 4, claim 5, claim 6, claim 7 and claim 8, wherein:
the housing assembly further includes:
a power-adjustment switch for a respective one of the tattoo machines to be respectively received and supported by the cradle assemblies; and
the power-adjustment switch configured to be selectively adjusted by a user in such a way that an amount of power to be consumed by a selected tattoo machine is adjustable.

10. The apparatus of any one of claim 3, claim 4, claim 5, claim 6, claim 7, claim 8 and claim 9, wherein:
the housing assembly further includes a power switch for a respective one of the tattoo machines to be respectively received and supported by the cradle assemblies; and
the power switch is configured to selectively turn ON or turn OFF power to be made available to a selected one of the tattoo machines.

11. The apparatus of any one of claim 3, claim 4, claim 5, claim 6, claim 7, claim 8, claim 9 and claim 10, wherein:
the cradle assemblies are configured to be removable from a respective pressure switch.

12. The apparatus of any one of claim 3, claim 4, claim 5, claim 6, claim 7, claim 8, claim 9, claim 10 and claim 11, wherein:
the housing assembly further includes:
a foot-switch input configured to be electrically connected to a plug of a foot-switch wire; and
the foot-switch wire configured to be electrically connected to a foot-activated switch; and
the foot-activated switch configured to:
turn OFF the electrical power that is provided to a selected one of the tattoo machines, and
turn OFF the electrical power in such a way that no electrical power is provided to any one of the tattoo machines.

13. The apparatus of claim 12, wherein:
the housing assembly further includes:
a controller being electrically connected to a power display unit, a power-adjustment switch, and a power switch; and
the controller being electrically connected to a power output, in which the power output is configured to be electrically connected to a tattoo machine; and
the controller being configured to be electrically connectable to any one of:
a power-cord input, which is electrically connected to a power cord; and
the power output, which is electrically connected to an external power supply; and
the controller being electrically connected to the foot-switch input, in which the foot-switch input is configured to be electrically connected to the foot-switch wire of the foot-activated switch.

14. The apparatus of claim 13, wherein:
the foot-activated switch is configured to be closed in response to a foot of a user depressing the foot-activated switch which, in this case, indicates a power ON request to the controller via the foot-switch wire, and for the case where the foot-activated switch is depressed by the foot of the user, the foot-activated switch, in use, provides a first indication to the controller that a user desires application of electrical power to be connected to the tattoo machine.

15. The apparatus of any one of claim 13 and claim 14, wherein:
the foot-activated switch is configured to be open in response to a foot of a user not depressing the foot-activated switch which, in this case, indicates a power OFF request to the controller via the foot-switch wire and for the case where the foot-activated switch is not depressed by the foot of the user, the foot-activated switch, in use, provides a second indication to the controller that the user does not desire an application of electrical power to the tattoo machine.

16. The apparatus of any one of claim 13, claim 14 and claim 15, wherein:
the controller is electrically connected to a pressure switch; and
the pressure switch is configured to transmit a first signal to the controller, in which the first signal indicates that the tattoo machine is received and supported by the cradle assembly; and
the controller is configured to not connect electrical power to the tattoo machine for the case where the controller receives the first signal from the pressure switch for the case where the tattoo machine is received and supported by the cradle assembly.

17. The apparatus of any one of claim 13, claim 14, claim 15 and claim 16, wherein:
the controller is electrically connected to the pressure switch; and
the pressure switch is configured to:
detect that the tattoo machine has become removed from the cradle assembly, for the case where a user removes the tattoo machine from the cradle assembly, and away from the housing assembly; and
transmit a second signal to the controller, in which the second signal indicates that the tattoo machine has become removed from the cradle assembly.

18. The apparatus of any one of claim 13, claim 14, claim 15, claim 16 and claim 17, wherein:
the controller is electrically connected to a pressure switch; and
the controller is configured to not connect electrical power to the tattoo machine for the case where the controller, in use:
receives a first signal from the pressure switch for the case where the tattoo machine is, at least in part, received and supported by the cradle assembly; and
receives a second indication from the foot-activated switch, in which a user does not desire an application of electrical power to the tattoo machine.

19. The apparatus of any one of claim 13, claim 14, claim 15, claim 16, claim 17 and claim 18, wherein:
the controller is electrically connected to a pressure switch; and
the controller is configured to connect electrical power to the tattoo machine for the case where the controller, in use:
receives a second signal from the pressure switch for the case where the tattoo machine is not received and supported by the cradle assembly; and
receives a first indication from the foot-activated switch, in which a user desires an application of electrical power to be connected to the tattoo machine.
